# EUROPEAN PATENT SPECIFICATION

(11) **EP 1 289 433 B1**
(45) Date of publication and mention of the grant of the patent: **07.01.2009**
(21) Application number: 01934207.0
(22) Date of filing: 06.06.2001
(51) Int. Cl.: A61B 17/28, B26B 13/28

(54) **HAND HELD SURGICAL INSTRUMENT**
IN DER HAND HALTBARES CHIRURGISCHES WERKZEUG
INSTRUMENT CHIRURGICAL MANUEL

(30) Priority: 10.06.2000 GB 0014120
(43) Date of publication of application: 12.03.2003
(73) Proprietor: Sinton, Richard Thompson, Melrose TD6 9AH (GB)
(72) Inventor: Sinton, Richard Thompson, Melrose TD6 9AH (GB)
(74) Representative: Szczuka, Jan Tymoteusz
(86) International application number: PCT/GB2001/002474
(87) International publication number: WO 2001/095812

(56) References cited:
- DE-C- 98 306
- DE-U- 29 712 016
- GB-A- 2 280 397

## Description

The present invention relates to a hand instrument and to a method of sterilisation thereof. The instrument is suitable for use as a pincer instrument such as forceps, needle holders, scissors and clamps and the invention relates particularly to surgical instruments.

A large range of well-known hand instruments such as needleholders, scissors, forceps, tongs and the like operate using a pincer movement. Hand instruments of the foregoing type typically comprise two limbs pivotally joined by a simple screw or rivet arrangement, or they may be joined by a "box joint" arrangement wherein part of one limb passes through a widened hollow portion of the other limb.

An inherent problem with all of the above-mentioned instruments is that it is difficult to clean in and around the pivotal connection area thereof. The difficulty in cleaning arises as a result of the nature of the pivotal connection wherein the limbs have sliding contact surfaces which are in substantially continuous intimate contact with each other. In use of the instruments particularly those found in use in surgery or food preparation, the sliding contact surfaces may be exposed to blood, food or other contaminant matter. As a result of such exposure, the instruments must be cleaned (i.e. sterilised) effectively before they can safely be re-used. This is especially critical for surgical and, to a lesser extent, food preparation applications.

The sterilisation of hand instruments for use in surgical procedures in human and non-human subjects is of ever increasing importance, especially in view of the continuing emergence of new and ever more dangerous pathogens which cause conditions such as for example new variant CJD, HIV, MRSA (methicillin resistant Staphylococcus Aureus) and necrotising fasciitis.

Instruments which have been used in surgical procedures may be exposed to a wide range of pathogens and infection carrying vectors which may be passed from patient to patient if the instruments are not completely and efficiently sterilised between surgical procedures.

Presently, the most common means of sterilisation is by use of an autoclave, wherein surgical instruments and the like are exposed to intense heat and high pressures for a pre-determined period of time. The efficiency of such sterilisation techniques is dependant upon *inter alia* exposing all surfaces of the hand instrument (or other article to be cleaned) to the heat and pressure generated by the autoclave apparatus.

In addition to, or as an alternative to use of an autoclave, the instrument may be immersed in a cleaning medium with the limbs in a first open condition, then re-immersed in the cleaning medium in at least a second open position to expose as much of the sliding contact surfaces (and other portions of the instrument) to the cleaning medium as possible. The cleaning medium may be heated water containing one or more disinfecting/sterilising agents such as for example, fungicides, bactericides, sodium hypochlorite, etc.

Generally, however, even such measures are not sufficient to expose substantially all of the surface of a said instrument to the cleaning medium.

Moreover, those instruments provided with a box joint arrangement present a greater cleaning problem in that each limb portion has at least two sliding contact surfaces to be cleaned, as opposed to the usual one sliding contact surface per limb portion of instruments having only a simple pivot pin arrangement. Box joints are particularly preferred for use by surgeons for surgical purposes because of the friction joint between the limbs allowing the limbs to retain a fixed opening but their construction does not facilitate their economic or easy dismantling for cleaning and sterilising purposes.

In recognition of the abovementioned problems United Kingdom Patent Application Publication No.GB2280397 A provided for a hand instrument having limb portions which could be disconnected when in a non-use condition, thereby allowing full exposure of the sliding contact surfaces of the limbs and to allow effective cleaning thereof. The instrument of GB2280397 A and as described therein with reference to Fig.1 comprised *inter alia* a modified box joint wherein a first limb portion is provided with an opening formed and arranged for engaging or disengaging a pin member located on a second limb member when the first and second limb members are rotated relative one another about the box joint to a pre-determined release angle, e.g. to an angle of about 85° to 95°. The instrument of GB2280397 A has the disadvantage that if the limb portions are intentionally or unintentionally opened to the pre-determined release angle, then the limb portions simply disengage from one another. This is highly undesirable, especially during a surgical procedure.

Similarly, other instruments of the prior art (see for example, GB966,058 A and GB698,921 A) each feature pivotally connected limb portions which are mutually disengagable from each other. The limb portions can be disconnected when they are rotated related to each other to a pre-determined release angle and the respective limb portions moved apart along a pivotal axis of the pivot thereof.

Again, as with the instrument of GB2280397 A, the instruments of the prior art suffer from *inter alia* the same disadvantage thereof, in that if the limb members are intentionally or unintentionally rotated relative each other to the pre-determined release angle, then the limb members will simply disengage from one another.

It is an object of the present invention to overcome or minimise one or more of the disadvantages of the prior art.

The present invention provides a scissor-action hand instrument (1) having first and second limbs (2, 4) pivotally connected together in between their ends, wherein the pivotal connection is of a so-called box-joint type wherein the first limb (2) has a tenon portion (14) and the second limb (4) has a mortice portion (16) through which said tenon portion (14) extends, said second limb (4) having at said mortice portion (16) a pivot pin (18) having a neck portion (20) and an enlarged head portion (22), said tenon portion (14) having a keyhole aperture (24) with a first reduced diameter portion (26) through which said pivot pin neck portion (20) is disposable in use of the instrument (1), and a second enlarged diameter portion (28) spaced apart from and connected to said first reduced diameter portion (26) and through which said pivot pin head portion (22) is passable for assembly and disassembly of the limbs (2, 4), the pivot pin neck portion (20) being translatable between said first and second aperture portions (26, 28), said mortice portion (16) having a central portion opening (30) at one side around said pivot pin head portion (22) which central portion opening (30) is formed and arranged for allowing passage therethrough of said tenon portion (14) only when said instrument (1) is in an open position with said limbs (2, 4) extending transversely of each other and the pivot pin head portion (22) and enlarged diameter portion (28) of the aperture (24) are in register with each other for passage of said pivot pin head portion (22) through said enlarged diameter portion (28) of the aperture (24) whereby said limbs (2, 4) are captively retained together in use of the instrument (1) with the pivot pin neck portion (20) disposed through said first reduced diameter aperture portion (26), and cannot be disengaged from each other until the limbs (2, 4) are subject to relative translational displacement to said open position so as to bring said pivot pin neck portion (20) into said second enlarged diameter aperture portion (28).

Advantageously, the pivot pin neck portion has a reduced thickness along an axis extending transversely of the principal plane of said mortice portion, and said first and second aperture portions are connected by a reduced width connection portion so that said pivot pin neck portion can only pass along said aperture connecting portion when said instrument is in a substantially open position with said limbs extending substantially transversely of each other.

Preferably the mortice and tenon portions are formed and arranged so that the opposed faces thereof are in substantial frictional inter-engagement with each other when said instrument is in a partly open position, said frictional inter-engagement being at least sufficient to retain the relative disposition of said limbs against the forces of gravity acting on an unsupported one of said limbs.

Preferably the arrangement of the mortice and tenon portions is formed and arranged with frictional inter-engagement to hold the first limb over a range of angles in the range of from 0° (jaws closed) to 80° (jaws open).

Preferably there is provided a resilient biasing means formed and arranged in use to bias the first and second limbs so that the respective jaw portions of each limb are pivotally spaced apart. The resilient biasing means may comprise a sprung member which may be secured to one or other of the limbs and formed and arranged in use to bear against the other of the limbs.

Preferably there is provided a limiting means formed and arranged in use to limit the extent of pivotal movement between the limbs. Desirably said limiting means may comprise an elongate member which is secured to one of the limbs and which co-operates with a stop portion on the other of the limbs to limit the pivotal movement of the limbs. Conveniently the limiting means may be formed and arranged so as to be disengageable.

The instrument may be made of any suitable material though for use in surgical applications steel, desirably stainless steel is preferred. The resilient biasing means may also desirably be made of steel.

The present invention also provided a method of sterilising a hand instrument of the present invention comprising the steps of:
providing a hand instrument according to the present invention;
disengaging said first and second limbs from one another; placing the disengaged limbs in a sterilising environment for a pre-determined period of time; and
removing the limbs from the sterilising environment.

Preferably said method includes a further step, prior to placing in a sterilising environment, of cleaning by scrubbing.

Preferably said sterilising environment is in the form of an autoclave.

Further preferred features and advantages of the present invention will appear form the following detailed description given by way of example of a preferred embodiment illustrated by reference to the accompanying drawings in which;
Fig. 1 is a plan view of a surgical instrument according to the present invention;

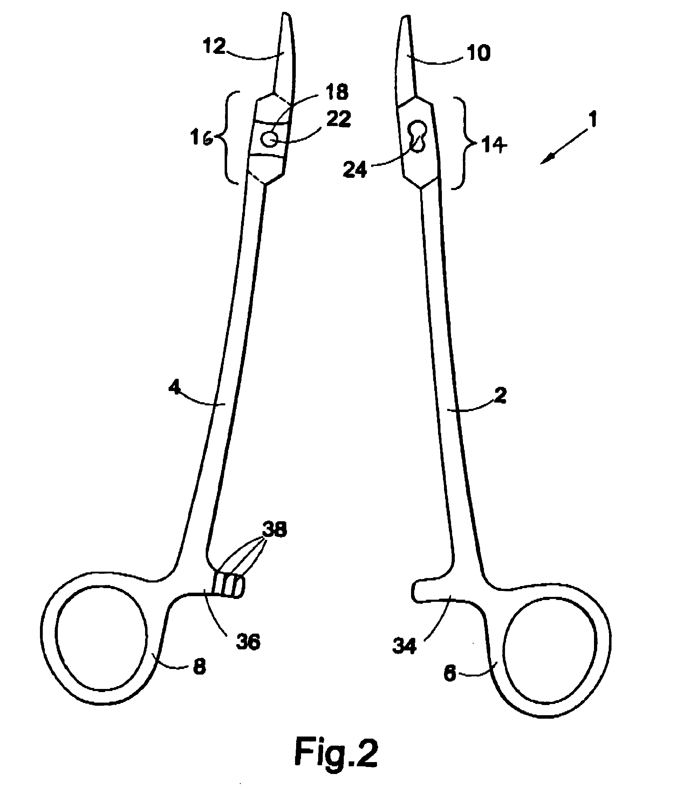

Fig. 2 is the plan view of the surgical instrument shown in Fig. 1 with the limbs disconnected;
Fig. 3 is a side view showing the limbs of the surgical instrument;
Fig. 4 a to c show respectively a underside view of the pivot pin, a left side view of the pivot pin, and a plan view of the pivot pin of the surgical instrument; and
Fig. 5 shows in more detail the aperture in one of the limbs of the surgical instrument.

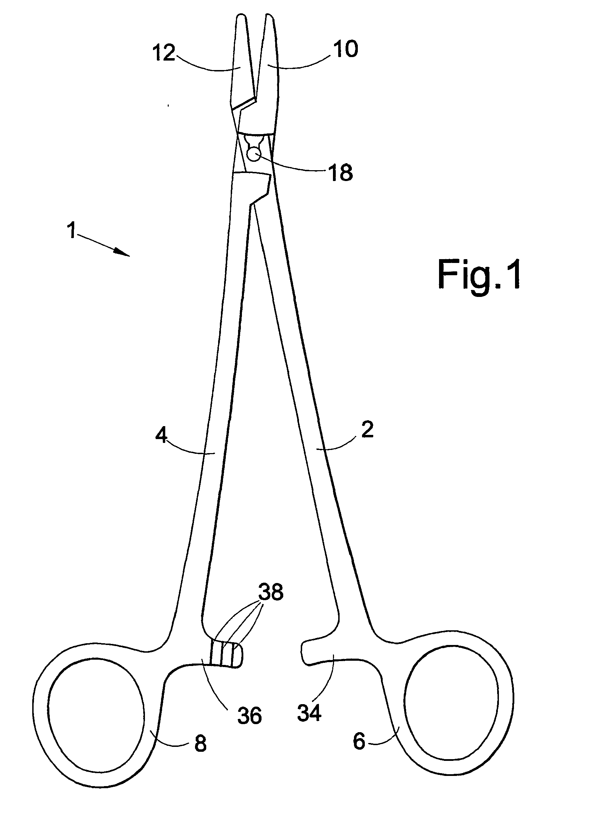

A surgical instrument, generally indicated by reference number 1 and shown in Fig. 1, comprises first and second limbs 2,4 pivotally connected together. Each limb has at one end a finger engagement aperture 6,8 and at the other end a jaw portion 10,12. The first and second limbs 2,4 are pivotally connected together between the ends.

The pivotal connection is in the form of a "box type joint" and the first limb 2 has a tenon portion 10 which co-operates with a mortice portion 16 on the second limb 4. The tenon portion 14 extends through the mortice portion 16.

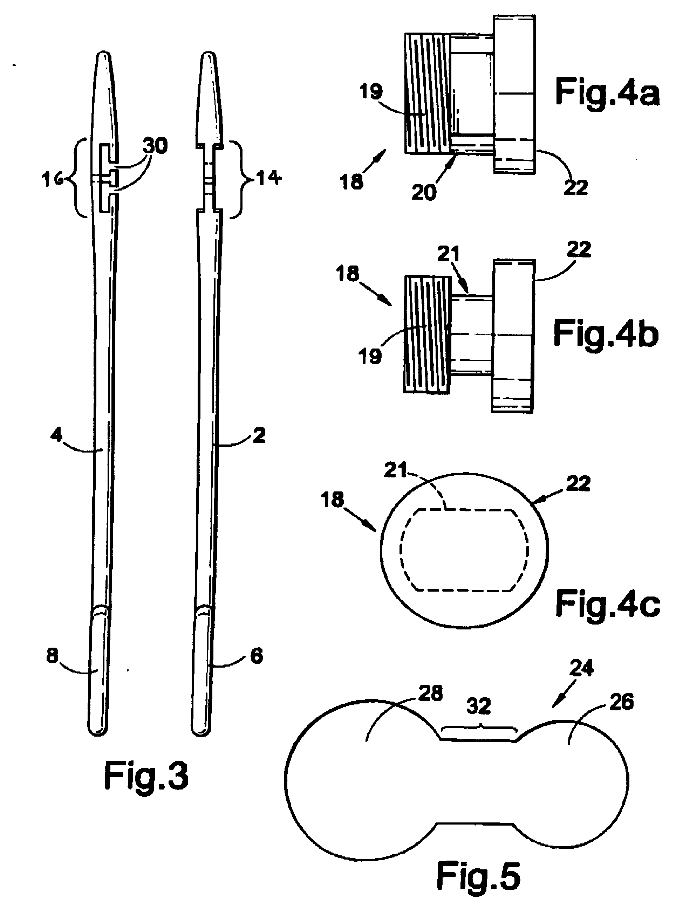

At the centre of the mortice portion 16 of the second limb 4 there is a pivot pin 18 which is screwably 19 attached into the second limb 4. With reference to Figs. 4 a to c the pivot pin 18 has a neck portion 20 and an enlarged head portion 22.

At the tenon portion 14 of the first limb 2 there is a keyhole aperture 24 having an hourglass shape (shown in more detail with reference to Fig. 5 of the accompanying drawings). The keyhole aperture 24 has a first reduced diameter portion 26 and a second enlarged diameter portion 28. The first reduced diameter portion 26 is of a diameter approximately equal to the diameter of the neck portion 20 of the pivot pin 18 such that pivot pin 18 is rotatable within the first reduced diameter portion 26. The second enlarged diameter portion 28, which is spaced apart from and connected to the first reduced diameter portion 26 is of a diameter approximately equal to the head portion 22 of the pivot pin 18 such that the head 22 of the pivot pin 18 may be passed through for assembly and disassembly of the limbs 2,4 of the instrument 1.

The passage between the first reduced diameter portion 26 and the second enlarged diameter portion 28 of the aperture 24 is such that the pivot pin neck portion 20 may pass along.

The mortice portion 16 of the second limb 4 has a central portion opening 30 at one side around the pivot pin head portion 22 which allows for passage of the tenon portion 14 of the first limb 2 when the instrument 1 is in a open position with the limbs 2,4 at approximately 90° to each other (not shown). The central portion 30 opening on the mortice portion 16 is just slightly wider than the width of the tenon portion 14 on the first limb 2. This arrangement is such that the limbs 2,4 may be assembled and disassembled only with the first and second limbs 2,4 arranged substantially transversely or at 90° to each other, that is, in a condition in which they would not normally be found in normal surgical use. When the first and second limbs 2,4 are in this transverse or 90° position then the pivot pin head portion 22 and the enlarged diameter portion 28 of the aperture are in register with each other in such a way that the pivot pin head portion 22 may pass through the enlarged diameter portion of the aperture 24 such that the limbs 2,4 are captively retained together with the pivot pin neck portion 20 disposed through the first reduced diameter portion 26 in such a way that they cannot be disengaged from each other until the limbs 2,4 are subjected to relative translational displacement so as to bring the pivot pin neck portion 20 into register with the second enlarged diameter aperture 28.

In more detail and with particular reference to Figs. 4 a to c and Fig. 5 it will be noted that the pivot pin neck portion 20 has a reduced thickness 21 along an axis extending transversely of the principal plane of the mortice portion 16 in such a way that the first and second aperture portions 26,28 are connected by a reduced width connecting portion 32 so that the pivot pin neck portion 20 can only pass along the passage connecting the first reduced diameter portion 26 and the second enlarged diameter portion 28 of the aperture 24 when then limbs 2,4 of the instrument 1 are in the above noted transversely or 90° position.

In further detail and with particular reference to Fig. 3 the mortice 16 and tenon 14 portions of the first 2 and second limbs 4 are formed and arranged in such a way that the opposed faces are in frictional interconnection with each other when the instrument 1 is in a partly open position. This frictional inter-engagement is at least sufficient to retain the relative disposition of the limbs 2,4 with respect to one another against the forces of gravity acting on the unsupported one of the limbs. This is particularly desirable in surgical procedures where a surgeon will expect to have an instrument 1 presented to him by a nurse with the instrument 1, or the jaws 10,12 thereof, open at a predetermined or given opening.

Adjacent the finger portion 6,8 of each of the limbs 2,4 there is a lug 34,36, each provided with a set of ratchet teeth 38 formed to co-operate with each other so that when the first and second lugs (34,36) engage with each other the ratchet teeth 38 interengage thereby locking the surgical instrument 1 in a closed condition (not shown).

Various modifications may be made to the above-described embodiment without departing from the scope of the present invention. Thus for example there may be provided a biasing arm mounted on the first limb, which arm bears against the second limb to maintain the jaws of the instrument slightly apart. To limit the extent to which the limbs may be pivotally moved apart, a limiting peg may be provided on the first limb which peg engages with a slot in an end of the biasing arm. It will be understood by those skilled in the art that although the above-described invention relates to a surgical instrument invention, it is also equally applicable to other types of instruments which employ a pivotal connection.

## Claims

1. A scissor-action hand instrument (1) having first and second limbs (2, 4) pivotally connected together in between their ends, wherein the pivotal connection is of a so-called box-joint type wherein the first limb (2) has a tenon portion (14) and the second limb (4) has a mortice portion (16) through which said tenon portion (14) extends, said second limb (4) having at said mortice portion (16) a pivot pin (18) having a neck portion (20) and an enlarged head portion (22), said tenon portion (14) having a keyhole aperture (24) with a first reduced diameter portion (26) through which said pivot pin neck portion (20) is disposable in use of the instrument (1), and a second enlarged diameter portion (28) spaced apart from and connected to said first reduced diameter portion (26) and through which said pivot pin head portion (22) is passable for assembly and disassembly of the limbs (2, 4), the pivot pin neck portion (20) being translatable between said first and second aperture portions (26, 28), said mortice portion (16) having a central portion opening (30) at one side around said pivot pin head portion (22) which central portion opening (30) is formed and arranged for allowing passage therethrough of said tenon portion (14) only when said instrument (1) is in an open position with said limbs (2, 4) extending transversely of each other and the pivot pin head portion (22) and enlarged diameter portion (28) of the aperture (24) are in register with each other for passage of said pivot pin head portion (22) through said enlarged diameter portion (28) of the aperture (24) whereby said limbs (2, 4) are captively retained together in use of the instrument (1) with the pivot pin neck portion (20) disposed through said first reduced diameter aperture portion (26), and cannot be disengaged from each other until the limbs (2, 4) are subject to relative translational displacement to said open position so as to bring said pivot pin neck portion (20) into said second enlarged diameter aperture portion (28).

2. A hand instrument (1) as claimed in claim 1 wherein the pivot pin neck portion (20) has a reduced thickness (21) along an axis extending transversely of the principal plane of said mortice portion (16), and said first and second aperture portions (26, 28) are connected by a reduced width connection portion (32) so that said pivot pin neck portion (20) can only pass along said aperture connecting portion when said instrument (1) is in a substantially open position with said limbs (2, 4) extending substantially transversely of each other.

3. A hand instrument (1) as claimed in claim 1 or claim 2 wherein, the mortice (16) and tenon portions (14) are formed and arranged so that opposed faces thereof are in substantial frictional inter-engagement with each other when said instrument (1) is in a partly open position, said frictional inter-engagement being at least sufficient to retain the relative disposition of said limbs (2, 4) against the forces of gravity acting on an unsupported one of said limbs (2, 4).

4. A hand instrument (1) as claimed in any one of claims 1 to 3 wherein, the arrangement of the mortice (16) and tenon portions (14) is formed and arranged with frictional inter-engagement to hold the first and second limbs (2, 4) in a pre-determined position relative to one another over a range of angles of from 0° to 80°.

5. A hand instrument (1) as claimed in any one of claims 1 to 4 wherein there is provided a resilient biasing means formed and arranged in use to bias the first and second limbs (2, 4) so that respective jaw portions (10, 12) of each limb (2, 4) are pivotally spaced apart.

6. A hand instrument (1) as claimed in claim 5 wherein the resilient biasing means comprises a sprung member which is secured to one or other of the limbs (2, 4) and formed and arranged in use to bear against the other of the limbs (2, 4).

7. A hand instrument (1) as claimed in any one of claims 1 to 6 wherein there is provided a limiting means formed and arranged in use to limit the extent of pivotal movement between the limbs wherein said limiting means comprises an elongate member which is secured to one of the limbs and which co-operates with a stop portion on the other of the limbs to limit the pivotal movement of the limbs (2, 4).

8. A hand instrument (1) as claimed in claim 7 wherein said limiting means is formed and arranged so as to be disengageable.

9. A hand instrument (1) as claimed in any one of claims 1 to 8 made from material selected from the group including steel and stainless steel.

10. A method of sterilising a hand instrument according to claim 1 comprising the steps of:
providing a hand instrument (1) according to claim 1; disengaging said first and second limbs (2, 4) from one another;
placing the disengaged limbs (2, 4) in a sterilising environment for a pre-determined period of time; and
removing the limbs from the sterilising environment.

11. A method as claimed in claim 10 which includes an initial step, prior to placing in a sterilising environment, of cleaning the hand instrument (1) by scrubbing.

12. A method as claimed in either of claims 10 and 11 wherein said sterilising environment is in the form of an autoclave.

## Patentansprüche

1. Handinstrument (1) mit Scherenwirkungsweise, das einen ersten und einen zweiten Schenkel (2, 4) hat, die zwischen ihren Enden schwenkbar miteinander verbunden sind, wobei die Schwenkverbindung von der sogenannten Buchsengelenkart ist, wobei der erste Schenkel (2) einen Zapfenabschnitt (14) hat und der zweite Schenkel (4) einen Zapfenlochabschnitt (16) hat, durch den sich der Zapfenabschnitt (14) erstreckt, wobei das zweite Glied (4) an dem Zapfenlochabschnitt (16) einen Gelenkzapfen (18) hat, der einen Halsabschnitt (20) und einen erweiterten Kopfabschnitt (22) hat, wobei der Zapfenabschnitt (14) eine Schlüssellochöffnung (24) mit einem ersten Abschnitt (26) mit verringertem Durchmesser, durch den der Gelenkzapfen-Halsabschnitt (20) bei Anwendung des Instruments (1) angeordnet werden kann, und einen zweiten Abschnitt (28) mit vergrößertem Durchmesser, der mit Zwischenraum zu dem ersten Abschnitt (26) mit verringertem Durchmesser angeordnet und mit demselben verbunden ist und durch den der Gelenkzapfen-Kopfabschnitt (22) zum Zusammenbauen und Auseinanderbauen der Schenkel (2, 4) hindurchgeführt werden kann, hat, wobei der Gelenkzapfen-Halsabschnitt (20) zwischen dem ersten und dem zweiten Öffnungsabschnitt (26, 28) verschoben werden kann, wobei der Zapfenlochabschnitt (16) eine Mittelabschnittsöffnung (30) auf einer Seite um den Gelenkzapfen-Kopfabschnitt (22) hat, wobei die Mittelabschnittsöffnung (30) dafür geformt und angeordnet ist, einen Durchgang des Zapfenabschnitts (14) durch dieselbe nur dann zu ermöglichen, wenn sich das Instrument (1) in einer offenen Position befindet, wobei sich die Schenkel (2, 4) quer zueinander erstrecken und sich der Gelenkzapfen-Kopfabschnitt (22) und der Abschnitt (28) mit vergrößertem Durchmesser der Öffnung (24) für einen Durchgang des Gelenkzapfen-Kopfabschnitts (22) durch den Abschnitt (28) mit vergrößertem Durchmesser der Öffnung (24) in Deckung befinden, wodurch die Schenkel (2, 4) bei Anwendung des Instruments (1) unverlierbar zusammengehalten werden, wobei der Gelenkzapfen-Halsabschnitt (20) durch den ersten Öffnungsabschnitt (26) mit verringertem Durchmesser angeordnet ist, und nicht voneinander ausgerückt werden können, bis die Schenkel (2, 4) einer relativen Translationsverschiebung zu der offenen Position unterworfen werden, um so den Gelenkzapfen-Halsabschnitt (20) in den zweiten Öffnungsabschnitt (28) mit vergrößertem Durchmesser zu bringen.

2. Handinstrument (1) nach Anspruch 1, wobei der Gelenkzapfen-Halsabschnitt (20) längs einer Achse, die sich quer zu der Hauptebene des Zapfenlochabschnitts (16) erstreckt, eine verringerte Dicke (21) hat und der erste und der zweite Öffnungsabschnitt (26, 28) durch einen Verbindungsabschnitt (32) mit verringerter Breite verbunden sind, so dass der Gelenkzapfen-Halsabschnitt (20) nur dann längs des Öffnungsverbindungsabschnitts hindurchgehen kann, wenn sich das Instrument (1) in einer im Wesentlichen offenen Position befindet, wobei sich die Schenkel (2, 4) im Wesentlichen quer zueinander erstrecken.

3. Handinstrument (I) nach Anspruch 1 oder Anspruch 2, wobei der Zapfenloch-(16) und der Zapfenabschnitt (14) so geformt und angeordnet sind, dass sich einander gegenüberliegende Flächen derselben in einem im Wesentlichen reibschlüssigen gegenseitigen Eingriff miteinander befinden, wenn sich das Instrument (1) in einer teilweise offenen Position befindet, wobei der reibschlüssige gegenseitige Eingriff wenigstens ausreichend ist, um die relative Anordnung der Schenkel (2, 4) gegen die Schwerkraft zu halten, die auf einen ungestützten der Schenkel (2, 4) einwirkt.

4. Handinstrument (1) nach einem der Ansprüche 1 bis 3, wobei die Anordnung des Zapfenloch- (16) und des Zapfenabsehnitts (14) mit einem reibschlüssigen gegenseitigen Eingriff geformt und angeordnet ist, um den ersten und den zweiten Schenkel (2, 4) über einen Bereich von Winkeln von 0° bis 80° in einer vorbestimmten Position im Verhältnis zueinander zu halten.

5. Handinstrument (1) nach einem der Ansprüche 1 bis 4, wobei ein elastisches Vorspannmittel bereitgestellt wird, das dafür geformt und angeordnet ist, bei Anwendung den ersten und den zweiten Schenkel (2, 4) so vorzuspannen, dass jeweilige Backenabschnitte (10, 12) jedes Schenkels (2, 4) schwenkend mit Zwischenraum zueinander angeordnet sind.

6. Handinstrument (1) nach Anspruch 5, wobei das elastische Vorspannmittel ein gefedertes Element umfasst, das an dem einen oder dem anderen der Schenkel (2, 4) befestigt und dafür geformt und angeordnet ist, bei Anwendung an dem anderen der Schenkel (2, 4) aufzuliegen.

7. Handinstrument (1) nach einem der Ansprüche 1 bis 6, wobei ein Begrenzungsmittel bereitgestellt wird, das dafür geformt und angeordnet ist, bei Anwendung das Ausmaß der Schwenkbewegung zwischen den Schenkeln zu begrenzen, wobei das Begrenzungsmittel ein längliches Element umfasst, das an dem einen der Schenkel befestigt ist und das mit einem Anschlagabschnitt an dem anderen der Schenkel zusammenwirkt, um die Schwenkbewegung der Schenkel (2, 4) zu begrenzen.

8. Handinstrument (I) nach Anspruch 7, wobei das Begrenzungsmittel so geformt und angeordnet ist, dass es ausgerückt werden kann.

9. Handinstrument (1) nach einem der Ansprüche 1 bis 8, das aus einem Material hergestellt ist, das aus der Gruppe, die Stahl und rostfreien Stahl einschließt, ausgewählt ist.

10. Verfahren zum Sterilisieren eines Handinstruments (1) nach Anspruch 1, wobei das Verfahren die folgenden Schritte umfasst:
Bereitstellen eines Handinstruments (1) nach Anspruch 1;
Ausrücken des ersten und des zweiten Schenkels (2, 4) voneinander;
Anordnen der ausgerückten Schenkel (2, 4) in einer Sterilisierungsumgebung für einen vorbestimmten Zeitraum; und
Entnehmen der Schenkel aus der Sterilisierungsumgebung.

11. Verfahren nach Anspruch 10, die einen anfänglichen Schritt, vor dem Anordnen in einer Sterilisierungsumgebung, einschließt, das Handinstrument (1) durch Schrubben zu reinigen.

12. Verfahren nach einem der Ansprüche 10 und 11, wobei die Sterilisierungsumgebung die Form eines Autoklaven hat.

## Revendications

1. Instrument manuel à action de ciseaux (1), comportant des première et deuxième branches (2, 4) connectées de manière pivotante entre leurs extrémités, la connexion pivotante étant d'un type dit à joint enclenché, la première branche (2) comportant une partie à tenon (14) et la deuxième branche (4) comportant une partie de mortaise (16), à travers laquelle s'étend ladite partie de tenon (14), ladite deuxième branche (4) comportant au niveau de ladite partie de mortaise (16) un pivot (18) comportant une partie de col (20) et une partie de tête élargie (22), ladite partie de tenon (14) comportant une ouverture en trou de serrure (24) avec une première partie à diamètre réduit (26), à travers laquelle ladite partie de col du pivot (20) peut être passée lors de l'utilisation de l'instrument (1), et une deuxième partie à diamètre accru (28) espacée de ladite première partie à diamètre réduit (26) et connectée à celle-ci, à travers laquelle ladite partie de tête du pivot (22) peut passer en vue d'un assemblage et d'un désassemblage des branches (2, 4), la partie de col du pivot (20) pouvant effectuer un mouvement de translation entre lesdites première et deuxième parties d'ouverture (26, 28), ladite partie de mortaise (16) comportant une ouverture de la partie centrale (30) au niveau d'un côté entourant ladite partie de tête du pivot (22), ladite ouverture de la partie centrale (30) étant formée et agencée de sorte à permettre le passage de ladite partie de tenon (14) uniquement lorsque ledit instrument (1) se trouve dans une position ouverte, lesdites branches (2, 4) s'étendant transversalement l'une par rapport à l'autre, et la partie de tête du pivot (22) et la partie à diamètre accru (28) de l'ouverture (24) étant calées l'une par rapport à l'autre pour permettre le passage de ladite partie de tête du pivot (22) à travers ladite partie à diamètre accru (28) de l'ouverture (24), lesdites branches (2, 4) étant ainsi retenues et bloquées dans un état assemblé lors de l'utilisation de l'instrument (1) avec la partie de col du pivot (20) agencée à travers ladite première partie d'ouverture à diamètre réduit (26) et ne pouvant pas être dégagées l'une de l'autre jusqu'à ce que les branches (2, 4) effectuent un déplacement par translation relatif vers ladite position ouverte, pour déplacer ladite partie de col du pivot (20) dans ladite deuxième partie d'ouverture à diamètre accru (28).

2. Instrument manuel (1) selon la revendication 1, dans lequel la partie de col du pivot (20) a une épaisseur réduite (21) le long d'un axe s'étendant transversalement par rapport au plan principal de ladite partie de mortaise (16), lesdites première et deuxième parties d'ouverture (26, 28) étant connectées par une partie de connexion à largeur réduite (32), de sorte que ladite partie de col du pivot (20) ne peut passer le long de ladite partie de connexion d'ouverture que lorsque ledit instrument (1) se trouve dans une position pratiquement ouverte, lesdites branches (2, 4) s'étendant de manière pratiquement transversale l'une par rapport à l'autre.

3. Instrument manuel (1) selon les revendications 1 ou 2, dans lequel les parties de mortaise (16) et de tenon (14) sont formées et agencées de sorte que leurs faces opposées sont pratiquement engagées mutuellement par frottement lorsque ledit instrument (1) se trouve dans une position en partie ouverte, ledit engagement mutuel par frottement étant au moins suffisant pour retenir l'agencement relatif desdites branches (2, 4) contre la force de gravité agissant sur une branche non supportée desdites branches (2, 4).

4. Instrument manuel (1) selon l'une quelconque des revendications 1 à 3, dans lequel l'agencement des parties de mortaise (16) et de tenon (14) est formé et agencé par un engagement mutuel par frottement pour retenir les première et deuxième branches (2, 4) dans une position mutuelle prédéterminée, au-delà d'un intervalle d'angles allant de 0° à 80°.

5. Instrument manuel (1) selon l'une quelconque des revendications 1 à 4, comportant un moyen poussoir élastique formé et agencé de sorte à pousser en service les première et deuxième branches (2, 4), de sorte que les parties de mâchoire respectives (10, 12) de chaque branche (2, 4) sont espacées de manière pivotante.

6. Instrument manuel (1) selon la revendication 5, dans lequel le moyen poussoir élastique comprend un élément à ressort fixé sur l'une ou l'autre des branches (2, 4) et formé et agencé de sorte à reposer en service contre l'autre branche (2, 4).

7. Instrument manuel (1) selon l'une quelconque des revendications 1 à 6, comportant un moyen de limitation formé et agencé de sorte à limiter en service l'étendue du mouvement de pivotement entre les branches, le moyen de limitation comprenant un élément allongé fixé sur l'une des branches et coopérant avec une partie d'arrêt sur l'autre branche pour limiter le déplacement pivotant des branches (2, 4).

8. Instrument manuel (1) selon la revendication 7, dans lequel ledit moyen de limitation est formé et agencé de sorte à pouvoir être dégagé.

9. Instrument manuel (1) selon l'une quelconque des revendications 1 à 8, composé d'un matériau sélectionné dans le groupe constitué d'acier et d'acier inoxydable.

10. Procédé de stérilisation d'un instrument manuel selon la revendication 1, comprenant les étapes ci-dessous :
fourniture d'un instrument manuel (1) selon la revendication 1 ;
dégagement desdites première et deuxièmes branches (2,4) l'une de l'autre ;
placement des branches dégagées (2, 4) dans un environnement de stérilisation pour une période de temps prédéterminée ; et
retrait des branches de l'environnement de stérilisation.

11. Procédé selon la revendication 10, englobant l'étape initiale, avant l'étape de placement dans un environnement de stérilisation, de nettoyage de l'instrument manuel (1) par brossage.

12. Procédé selon l'une des revendications 10 ou 11, dans lequel ledit environnement de stérilisation a la forme d'un autoclave.
